## Europäisches Patentamt
## European Patent Office
## Office européen des brevets

(19)

(11) Publication number: **0 151 992**
**A2**

# (12) EUROPEAN PATENT APPLICATION

(21) Application number: **85100769.0**

(22) Date of filing: **25.01.85**

(51) Int. Cl.⁴: **A 61 K 7/48**

(30) Priority: **30.01.84 US 574927**

(43) Date of publication of application:
**21.08.85 Bulletin 85/34**

(84) Designated Contracting States:
**BE CH DE FR GB IT LI NL SE**

(71) Applicant: **MIRANOL CHEMICAL COMPANY, INC.**
**68 Culver Road P.O. Box 411**
**Dayton New Jersey(US)**

(72) Inventor: **Nadolsky, Richard J.**
**Island Spring Road**
**Clarksburg New Jersey(US)**

(72) Inventor: **Laryea, Joseph M.**
**14 Glen Avenue**
**Old Bridge New Jersey(US)**

(74) Representative: **Baillie, Iain Cameron et al,**
**c/o Ladas & Parry Isartorplatz 5**
**D-8000 München 2(DE)**

(54) Composition useful in cosmetics and toiletries.

(57) Partial esters of tripentaerythritol with from 1 to 7.5 mole of a saturated or unsaturated fatty acid of 12 to 20 carbon atoms per mole of tripentaerythritol have exceptional moisturizing and emollient properties which are of use in cosmetic compositions.

EP 0 151 992 A2

## COMPOSITION USEFUL IN COSMETICS AND TOILETRIES
### Background of the Invention
### 1. General Subject of the Invention

The present invention relates to a composition useful in cosmetics and toiletries and which contains partial esters of tripentaerythritol (2,2 bis [3-hydroxy-2,2,-bis-(hydroxymethyl) propoxymethyl]-1,3-propane diol).

### 2. Prior Art

Esters of tripentaerythritol have been known for many years and a variety of uses proposed for them. For example, (poly) pentaerythritols partially esterified with oleic acid were described as useful solvents in vapor-liquid chromatographic separation in U.S. Patent 3,358,423 issued to Emmanuel M. Amir on December 19, 1967. The use of a polyoxyethylene derivative of tripentaerythritol mono oleate as a viscosity control agent in polyvinyl chloride is described in U.S. Patent 2,657,186 issued on October 27, 1953 to David Y. Klein et al. Esters of tripentaerythritol with fatty acids of 4-8 carbon atoms were suggested by Hurwitz in U.S. Patent 2,958,706 (issued November 1, 1960) as being useful in plasticizing polyvinyl chloride. Partial esters of (poly) pentaerythritols with valeric acid are suggested in German Offenlegungsschrift 2226321 to be useful in the manufacture of lubricants.

The use of partial esters of tripentaerythritol with fatty acids of 12 to 18 carbon atoms wherein the degree of esterification is from 2 to 8 acid units as defoaming agents for use in latex paints is described in

CASE: U 4933

Silverstein et al's U.S. Patent 2,686,766 issued on August 17, 1954.

Conventional moisturizing compositions are typically based on glycerin which has an affinity for water and holds or attracts it to the skin. Emollients act to replenish skin oils and provide elasticity and smoothness. They are fatty materials, such as isopropyl myristate, isopropylpalmitate, acetylated lanolin alcohols and mineral oils.

Summary of the Invention

It is an object of the present invention to produce cosmetic and toiletery compositions and cosmetic treatments which utilize the surprising moisturizing and emollient properties we have found in certain partial esters of tripentaerythritol. These esters are readily absorbed in the skin and do not have a greasy feel. They provide a long-lasting emollient effect which may be noticeable even after bathing.

From a first aspect, the present invention provides a composition suitable for application to the skin which comprises an affective moisturizing or emollient amount of an ester of tripentaerythritol esterified with from 1 to 7.5 moles per mole of tripentaerythritol of saturated or unsaturated alkanoic acid of 12 to 20 for example 12 to 18 carbon atoms.

From a second aspect, the invention provides a cosmetic treatment which comprises administering to the skin a skin-moisturizing or emollient amount of a partial ester of tripentaerythritol esterified with from 1 to 7.5 moles of a saturated or unsaturated fatty acid of 12 tc 20 for example 12 to 18 carbon atoms.

DETAILED DESCRIPTION OF
THE INVENTION

Compositions according to the present invention are of use in a variety of circumstances wherein a moisturizing or emollient effect is desired. Typically, compositions according to the invention may be in a variety of forms including creams or lotions and solid forms such as bar soaps and lipsticks and comprise other cosmetically-acceptable materials such as emulsifiers, soaps or waxes in addition to the hereinbefore described partial esters of tripentaerythritol.

Cream and lotion formulations normally comprise emulsifiers. Suitable emulsifiers include amine salts of fatty acids of 12 to 20 carbon atoms, for example, alkanolamine salts (e.g. triethanolamine salts) of lauric, myristic, stearic and palmitic acids, mono esters of glycerol with fatty acids of 12 to 20 carbon atoms, such as glycerol monostearate, and poly alkoxylated fatty alcohols of 12 to 20 carbon atoms such as ethyoxylates, propoxylates and of oleyl alcohol and poly alkoxylate esters (such as poly ethoxylates and polypropoxylates) of fatty acids of 12 to 20 carbon atoms having from 1 to 20 alkylene oxide units. Such compositions may also contain humectants such as glycerol, sorbitol and $C_2-C_4$ alkylene glycols, such as propylene glycol.

Both creams and lotions according to the invention contain substantial quantities of water. The exact physical composition depending upon the ratio of emulsifier to water. We have found that compositions of the present invention require the presence of a lower amount of emulsifier than do prior art moisturizing compositions to ensure a stable cream form of the composition. Typically, skin lotion according to the present invention contain from 65 to 85 percent by weight water whereas creams contain less than 50 percent by weight water.

Compositions according to the invention may also contain further components such as emollients. For example, acetylated lanolin alcohols, isopropylmyristate or isopropyl stearate, propylene glycol dipelargonate and opacifiers and pigments such as kaoline and titanium dioxide.

Compositions of the present invention typically contain from 2 to 10 percent by weight of the specified partial ester of tripentaerythritol. Preferably from 2.5 to 5 percent by weight is composed of such a partial ester.

We have found that partial esters of tripentaerythritol containing from 4 to 6 ester groups are particularly useful. Preferred fatty acids with which tripentaerythritol is esterified are lauric, myristic, palmitic, stearic and oleic acids and mixtures of these. The mixtures of fatty acids commonly known as "coco" fatty acids may be used.

The partial esters of tripentaerythritol for use in this invention may be prepared by reacting from 1.0 to 7.5 moles of fatty acids with 1.0 mole of tripentaerythritol, in a suitable solvent that will azeotropically remove the water of reaction, and in the presence of a suitable acidic catalyst. Reactions are run at temperatures of 150-200°C. Suitable solvents are benzene, toluene, xylene or other solvent that will form an azeotrope with water and allow for achieving a reaction temperature in the range given above. Suitable catalysts are p-toluenesulfonic acids, stannous octoate, or other sufficiently thermally stable Lewis or Bronsted acids.

The invention will now be illustrated by the following Preparations and Examples:

PREPARATION 1:

To a flask equipped with thermometer, mechanical stirrer, reflux condenser and Dean-Stark trap were charged a blen (71/29) of lauric/myristic acid (124.8g, 0.6 mole) xylene (35g) and p-toluenesulfonic acid (0.38g). This was heated to 50 - 60°C. and tripentaerythritol(7.4g 0.2 mole) added portionwise, with good mixing to ensure that it is well dispersed. Heat to a maximum temperature of 190-195°C. while azeotropically removing water. Reaction was complete when the theoretical amount of water (10.8 ml) had been collected in the Dean-Stark trap. Reaction time was about three hours. When reaction was complete, the product was cooled to below 80°C. and 0.45g of a 25% solution of sodium methoxide in methanol added to neutralize the catalyst. The resulting product was heated under vacuum (>29 inches water) to distill off all the methanol and xylene. The product was cooled under vacuum to <80°C. before removing from the flask. When fully cooled, the product is a soft solid having an acid value <10. Conducting the reaction under an atmosphere of nitrogen gives a somewhat lighter-colored product.

PREPARATION 2:

A product was made by the same procedure as Preparation 1, except the mole ratio of lauric/myristic acid blend to tripentaerythritol was 4 to 1. This product was also a soft solid.

PREPARATION 3:

A product was made by the procedure of Preparation 1 using a 4 to 1 molar ratio of lauric/myristic acid blen to tripentaerythritol. Instead of p-toluenesulfonic acid, an equal amount of stannous octoate was used as catalyst. In this case, no neutralizing agent was added before removal of the xylene. This produced a material comparable to that obtained in Preparation 2, but of lighter color.

PREPARATION 4:

A product was made by the procedure of Preparation 1 using a 5 to 1 molar ratio of the same acid blend to tripentaerythritol. This product was a soft solid.

PREPARATION 5:

A product was made by the procedure of Preparation 1 using the same acid blend with tripentaerythritol at a respective molar ratio of 6 to 1. This product was a soft solid.

PREPARATION 6:

A product was made by the procedure of Preparation 1 using a 7.5 to 1 molar ratio of the same acid blend to tripentaerythritol. The product was a soft solid.

PREPARATION 7:

A product was made by the procedure of Preparation 1 using a 5 to 1 molar ratio of palmitic/stearic acid blend to tripentaerythritol. This product was a hard, wax-like solid.

PREPARATION 8:

A product was made by the procedure of Preparation 3 using a 5 to 1 molar ratio of lauric acid to tripentaerythritol. The product was a soft solid.

PREPARATION 9:

A product was made by the procedure of Preparation 3 using a 4 to 1 molar ratio of oleic acid to tripentaerythritol. The product was a viscous liquid.

Examples 1 - 4 and Comparative Test A

### SKIN LOTION

Skin lotions were prepared as follows:

|  | EXAMPLE 1 | EXAMPLE 2 | EXAMPLE 3 | EXAMPLE 4 | COMPARATIVE TEST A |
|---|---|---|---|---|---|
| I. Product from Preparation 2 | 5.0 | – | – | – | – |
| Product from Preparation 4 | – | 5.0 | – | – | – |
| Product from Preparation 5 | – | – | 5.0 | – | – |
| Product from Preparation 6 | – | – | – | 5.0 | – |
| Arlacel 165[1] (ICI Americas) | 6.5 | 6.5 | 6.5 | 6.5 | 6.5 |
| Acetol[2] (Emery Industries) | 3.5 | 3.5 | 3.5 | 3.5 | 3.5 |
| Petrolatum | 2.0 | 2.0 | 2.0 | 2.0 | 2.0 |
| Stearic Acid | 2.0 | 2.0 | 2.0 | 2.0 | 2.0 |
| Oleth-5[3] | 1.0 | 1.0 | 1.0 | 1.0 | 1.0 |
| Stearyl Alcohol | 0.5 | 0.5 | 0.5 | 0.5 | 0.5 |

Example 1 - 4 (con't)

| | | EXAMPLE 1 | EXAMPLE 2 | EXAMPLE 3 | EXAMPLE 4 | COMPARATIVE TEST A |
|---|---|---|---|---|---|---|
| II. | Propylene Glycol | 3.5 | 3.5 | 3.5 | 3.5 | 3.5 |
| | Water | 75.5 | 75.5 | 75.5 | 75.5 | 80.5 |
| | Triethanol-amine | 0.5 | 0.5 | 0.5 | 0.5 | 0.5 |

1) Glycerol monostearate and polyoxyethylene stearate

2) Acetylated lanolin alcohols

3) Ethoxylated oleyl alcohol

Procedure: I and II were in each case heated separately to 75°C. With good agitation, II was added to I. Agitation was continued until mixture is uniform and it was then allowed to cool.

Evaluation: A panel of 6 was asked to judge these products as hand lotions on the basis of texture, absorbability and feel on the skin. Significant preference was shown for the compositions of Examples 1 and 2 over those of Examples 3 and 4 and comparative test A with a slight preference for the product of Example 1 over that of Example 2.

Comments were that the products of Examples 1 and 2 gave a silky, non-greasy feel which some panelists claimed persisted even after washing.

Examples 5 and 6 and Comparative Tests B and C

SKIN LOTIONS

| | | EXAMPLE 5 | EXAMPLE 6 | COMPARATIVE TEST B | COMPARATIVE TEST C |
|---|---|---|---|---|---|
| I. | Product from Preparation 2 | 5.0 | - | - | - |
| | Product from Preparation 4 | - | 5.0 | - | - |

Example 5 and 6 (con't)

| | EXAMPLE 5 | EXAMPLE 6 | COMPARATIVE TEST B | COMPARATIVE TEST C |
|---|---|---|---|---|
| Dow Silicone Fluid 200 | – | – | – | 5.0 |
| Mineral Oil | 10.0 | 10.0 | 10.0 | 10.0 |
| Arlacel 165 | 6.5 | 6.5 | 6.5 | 6.5 |
| Isopropyl Myristate | 5.0 | 5.0 | 5.0 | 5.0 |
| Stearic Acid | 2.0 | 2.0 | 2.0 | 2.0 |
| Oleth-5 | 1.0 | 1.0 | 1.0 | 1.0 |
| Stearyl Alcohol | 0.5 | 0.5 | 0.5 | 0.5 |
| II. Propylene Glycol | 3.5 | 3.5 | 3.5 | 3.5 |
| Water | 66.0 | 66.0 | 71.0 | 66.0 |
| Triethanol-amine | 0.5 | 0.5 | 0.5 | 0.5 |

Procedure: Same as for Examples 1 - 4.

Evaluation: A panel of six chose the products of Examples 5 and 6 over the products of comparative tests B and C on the basis of absorbability and feel when tested as a hand lotion.

Examples 7 - 10 and Comparative Test D

SKIN LOTION

| | EXAMPLE 7 | EXAMPLE 8 | COMPARATIVE TEST D | EXAMPLE 9 | EXAMPLE 10 |
|---|---|---|---|---|---|
| I. Product from Preparation 2 | 5.0 | 2.5 | – | – | – |
| Product from Preparation 5 | – | – | – | 2.5 | – |
| Product from Preparation 1 | – | – | – | – | 5.0 |
| Super Sterol Ester (Croda) | – | – | 5.0 | – | – |
| Arlacel 165 | 6.5 | 6.5 | 6.5 | 6.5 | 6.5 |
| Acetol | 3.5 | 3.5 | 3.5 | 3.5 | 3.5 |
| Petrolatum | 2.0 | 2.0 | 2.0 | 2.0 | 2.0 |

Examples 7 - 10 (con't)

| | EXAMPLE 7 | EXAMPLE 8 | COMPARATIVE TEST D | EXAMPLE 9 | EXAMPLE 10 |
|---|---|---|---|---|---|
| Stearic Acid | 2.0 | 2.0 | 2.0 | 2.0 | 2.0 |
| Oleth-5 | 1.0 | 1.0 | 1.0 | 1.0 | 1.0 |
| II. Propylene Glycol | 3.5 | 3.5 | 3.5 | 3.5 | 3.5 |
| Water | 76.0 | 78.5 | 76.0 | 78.5 | 76.0 |
| Triethanol-amine | 0.5 | 0.5 | 0.5 | 0.5 | 0.5 |

Procedure: Same as for Examples 1 - 4.

Evaluation: A panel of seven favored the products of Examples 7, 8 and 9 over those of Comparative Test D and Example 10 by a factor of 5:2 when judging these formulas as hand lotions based on the criteria mentioned previously. Order of preference as follows: Example 7 > Example 8 > Example 9 > Example 10 > Comparative Test D

Examples 11 - 13 and Comparative Test E

CREAM FOR CHRONIC DRY SKIN

| | EXAMPLE 11 | COMPARATIVE TEST E | EXAMPLE 12 | EXAMPLE 13 |
|---|---|---|---|---|
| I. Product from Preparation 3 | 5.0 | – | 3.5 | 5.0 |
| Mineral Oil | 30.0 | 30.0 | 30.0 | 30.0 |
| Isopropyl Myristate | 5.0 | 5.0 | 5.0 | 5.0 |
| Arlacel 165 | 6.5 | 6.5 | 6.0 | 5.0 |
| Stearic Acid | 1.0 | 1.0 | 1.0 | 1.0 |
| Stearyl Alcohol | 0.5 | 0.5 | 0.5 | 0.5 |
| II. Water | 46.5 | 51.5 | 48.5 | 48.0 |
| Propylene Glycol | 5.0 | 5.0 | 5.0 | 5.0 |
| Triethanol-amine | 0.5 | 0.5 | 0.5 | 0.5 |

Procedure: Mixture I was heated to 80°C. and mixture

EXAMPLES 11 - 13 (con't)

II to 70°C. With agitation, II was added to I and agitation continued until uniform. The product was then allowed to cool.

Evaluation: The product of Example 11 performed better than that of Test E in all cosmetic parameters. A panel of five chose the product of Example 11 over that of Test E unanimously. Because Example 11 was too thick, it was reformulated to an acceptable cosmetic elegance in terms of appearance and rheology (Examples 12 and 13). A panel of five chose Example 12 over Example 13 (3:2) in terms of moisturization and non-greasy after feel.

### Example 14 and Test F
### MOISTURIZING LOTION

|      |                                        | EXAMPLE 14 | TEST F |
|------|----------------------------------------|------------|--------|
| I.   | Product from Preparation 3             | 5.0        | –      |
|      | Mineral Oil                            | 3.0        | 3.0    |
|      | Acetol                                 | 1.0        | 1.0    |
|      | Arlacel 165                            | 6.0        | 6.0    |
|      | Stearic Acid                           | 2.0        | 2.0    |
| II.  | Water                                  | 78.5       | 83.5   |
|      | Propylene Glycol                       | 3.5        | 3.5    |
|      | Veegum HV[1] (R. T. Vanderbilt)        | 0.5        | 0.5    |
|      | Triethanolamine                        | 0.5        | 0.5    |

[1] Colloidal magnesium aluminum silicate thickener

Procedure: Mixtures I and II were heated separately to 75°C. With agitation, II was added to I and agitation continued until uniform. The product was allowed to cool.

Evaluation: A panel of five chose Example 14 over Test F unanimously.

Observation: The product of Example 14 has better viscosity, lotionlike consistency and appearance than that of Test F.

### Examples 15-17 and Test G

|  |  | Example 15 | Test G | Example 16 | Example 17 |
|---|---|---|---|---|---|
| I. | Product from Preparation 3 | 4.0 | – | 4.0 | 4.0 |
|  | Mineral Oil | 15.0 | 15.0 | 7.5 | 3.5 |
|  | Isopropyl Myristate | 5.0 | 5.0 | 3.5 | 2.0 |
|  | Propylene Glycol Dipelargonate | 3.5 | 3.5 | 2.0 | 1.0 |
|  | Arlacel 165 | 5.0 | 5.0 | 5.0 | 5.0 |
|  | Beeswax | 2.0 | 2.0 | 2.0 | 2.0 |
|  | Stearic Acid | 1.0 | 1.0 | 1.0 | 1.0 |
|  | Stearyl Alcohol | 0.5 | 0.5 | 0.5 | 0.5 |
|  | Dow Corning Fluid 200 | 1.0 | 1.0 | 0.5 | 0.5 |
| II. | Water | 51.2 | 55.2 | 62.2 | 68.7 |
|  | Propylene Glycol | 3.5 | 3.5 | 3.5 | 3.5 |
|  | Carbopol 934 (3% Soln.) | 7.5 | 7.5 | 7.5 | 7.5 |
| III. | Triethanolamine | 0.8 | 0.8 | 0.8 | 0.8 |

Procedure:  Heat I to 75°C., II to 68°C.  With agitation add II to I.  Continue agitation until uniform.  Then add III.

Evaluation

1.  A panel of 5 chose Example 15 (4:1) over Test F, but found system of Example 15 to be very greasy. The composition was therefore reformulated using low levels of emollients.

2.  A panel of 4 chose Example 17 (4:0) over Example 16 in terms of moisturization, and found Example 16 to be greasy.

3.  A panel of 4 found Example 17 to provide excellent moisturization and a non-greasy after-feel.

Conclusion

The partial ester of pentaerythritol obtained by the process of preparation 3 can be used in combination Examples 15-17 (con't) with traditional emollients but is is desirable to reduce the levels of these emollients to avoid a greasy after feel as a result of excess emollients present in the formulation owing to the emollient properties of the pentaerythritol partial ester itself.

CLAIMS                                    0151992

1.      A composition suitable for topical application to
the skin which comprises an effective moisturizing or emollient amount
of an ester of tripentaerythritol esterified with from 1 to
7.5 moles per mole of tripentaerythritol of saturated or unsaturated alkanoic acid of 12 to 20 carbon atoms.

2.      A composition as claimed in claim 1, wherein the
tripentaerythritol ester is an ester wherein from 4 to 6 of
the hydroxy groups of tripentaerythritol have been esterified.

3.      A composition as claimed in either of claims 1 and 2,
wherein the alkanoic acid used is selected from the group
consisting of lauric, myristic, palmitic, stearic and oleic
acids and mixtures of these.

4.      A composition according to claim 3, wherein the tripentaerythritol ester is an ester wherein from 4 to 6 of the
hydroxy groups of tripentaerythritol have been esterified.

5.      A composition according to any one of the preceding
claims, which further contains a cosmetically-acceptable
emulsifier.

6.      A composition according to claim 5, wherein said
emulsifier is selected from the group consisting of amine
salts of fatty acids, monoesters of glycerol with a fatty
acid, fatty alcohols of 12 to 20 carbon atoms which are
optionally polyalkoxylated and polyalkoxylated esters of
fatty acids of 12 to 20 carbon atoms.

7.      A composition according to claim 6, which further
comprises an emollient.

8.      A composition according to claim 5, which further
comprises an emollient selected from the group comprising
acetylated lanolin alcohols, isopropylmyristate and
isopropylpalmitate and mineral oil.

9.      A composition according to claim 8, which is in the
form of a moisturizing lotion, an eye shadow preparation, a
moisturizing cream, bar soap, lipstick or makeup foundation.

10.     A cosmetic treatment which comprises administering to
the skin a skin-moisturizing or emollient amount of a partial
ester of tripentaerythritol esterified with from 1 to 7.5 moles
of saturated or unsaturated alkanoic acid of 12 to 20 carbon atoms.

CASE: U 4933